# FASCICULE DE BREVET EUROPEEN

(11) **EP 3 512 942 B1**
(45) Date de publication et mention de la délivrance du brevet: **25.11.2020**
(21) Numéro de dépôt: 17780799.7
(22) Date de dépôt: 12.09.2017
(51) Int. Cl.: C12N 9/88, C12P 19/12

(54) **NOUVELLE ULVANE LYASE ET SON UTILISATION POUR CLIVER DES POLYSACCHARIDES**
NEUARTIGE ULVANLYASE UND VERWENDUNG DAVON ZUR SPALTUNG VON POLYSACCHARIDEN
NOVEL ULVAN LYASE AND USE THEREOF FOR CLEAVING POLYSACCHARIDES

(30) Priorité: 13.09.2016 FR 1658505
(43) Date de publication de la demande: 24.07.2019
(73) Titulaire: Centre National de la Recherche Scientifique, 75016 Paris (FR); Sorbonne Université, 75006 Paris (FR)
(72) Inventeur: MICHEL, Gurvan, 29250 Plougoulm (FR); GUILLOUZO, Alexia, 28300 Champhol (FR); MASSÉ, Bertille, 29490 Guipavas (FR); GENICOT, Sabine, 29250 Saint-Pol-De-Léon (FR); GROISILLIER, Agnès, 29250 Sibiril (FR)
(74) Mandataire: Novagraaf Technologies
(86) Numéro de dépôt international: PCT/FR2017/052414
(87) Numéro de publication internationale: WO 2018/051002

(56) Documents cités:
- WO-A1-2011/157966
- WO-A1-2013/079828
- WO-A2-2009/016275
- DATABASE UniProtKB [Online] 13 novembre 2013 (2013-11-13), "SubName: Full=Ulvanlyase {ECO:0000313|EMBL:CDF79931.1};", XP002764322, extrait de Uniprot Consortium accession no. UNIPROT:T2KNC2 Database accession no. T2KNC2
- DATABASE ENA [Online] 31 août 2013 (2013-08-31), "Formosa agariphila KMM 3901 ulvanlyase", XP002764323, extrait de EBI accession no. EMBL:CDF79931 Database accession no. CDF79931
- DATABASE Protein [Online] 12 août 2015 (2015-08-12), "T9SS C-terminal target domain-containing protein [Formosa agariphila].", XP002764321, extrait de NCBI Database accession no. WP_038530530
- A. J. MANN ET AL: "The Genome of the Alga-Associated Marine Flavobacterium Formosa agariphila KMM 3901T Reveals a Broad Potential for Degradation of Algal Polysaccharides", APPLIED AND ENVIRONMENTAL MICROBIOLOGY, vol. 79, no. 21, 30 août 2013 (2013-08-30) , pages 6813-6822, XP055320131, US ISSN: 0099-2240, DOI: 10.1128/AEM.01937-13 cité dans la demande & DATABASE EMBL [Online] 31 août 2013 (2013-08-31), "Formosa agariphila KMM 3901, complete genome", extrait de EBI accession no. EM_STD:HG315671 Database accession no. HG315671
- MORAN KOPEL ET AL: "New Family of Ulvan Lyases Identified in Three Isolates from the Alteromonadales Order", JOURNAL OF BIOLOGICAL CHEMISTRY, vol. 291, no. 11, 13 janvier 2016 (2016-01-13), pages 5871-5878, XP055320498, US ISSN: 0021-9258, DOI: 10.1074/jbc.M115.673947

### SEQUENCE LISTING

<110> CENTRE NATIONAL DE LA RECHERCHE SCIENTIFIQUE - CNRS -
   UNIVERSITE PIERRE ET MARIE CURIE
   MICHEL, Gurvan
   GUILLOUZO, Alexia
   MASSE, Bertille
   GENICOT, Sabine
   GROISILLIER, Agnès
<120> NOUVELLE ULVANE LYASE ET SON UTILISATION POUR CLIVER DES POLYSACCHARIDES
<130> BNT221378PC00
<150> FR1658505
   <151> 2016-09-13
<160> 6
<170> PatentIn version 3.5
<210> 1
   <211> 1548
   <212> DNA
   <213> Formosa agariphila
<220>
   <221> CDS
   <222> (1)..(1548)
<400> 1
   atg cta gaa aaa act act ctt aaa aat att att tta ata cac ttt cta          48
   Met Leu Glu Lys Thr Thr Leu Lys Asn Ile Ile Leu Ile His Phe Leu
   1               5                   10                  15
   atg ttt tta gct gta gta aca gct caa acc gcg ccc gat gaa gat aca          96
   Met Phe Leu Ala Val Val Thr Ala Gln Thr Ala Pro Asp Glu Asp Thr
               20                  25                  30
   tct gct att aca aga tgt act gct gaa ggt aca aac cct gta aga gaa         144
   Ser Ala Ile Thr Arg Cys Thr Ala Glu Gly Thr Asn Pro Val Arg Glu
           35                  40                  45
   aca gat att cca aac ccc gta aat gtg gga act atc gat gac cgt agt         192
   Thr Asp Ile Pro Asn Pro Val Asn Val Gly Thr Ile Asp Asp Arg Ser
       50                  55                  60
   tgt tat gct aat tat aag gag agt aca gtt tat gga aaa act tgg ggt         240
   Cys Tyr Ala Asn Tyr Lys Glu Ser Thr Val Tyr Gly Lys Thr Trp Gly
   65                  70                  75                  80
   gtc tat aac ata acg ttt gat tct aat gat ttt gat acc tct tta caa         288
   Val Tyr Asn Ile Thr Phe Asp Ser Asn Asp Phe Asp Thr Ser Leu Gln
                   85                  90                  95
   cca aga ata gaa cgt tca tta agc cgc tcc agc gaa acg ggt att gga         336
   Pro Arg Ile Glu Arg Ser Leu Ser Arg Ser Ser Glu Thr Gly Ile Gly
               100                 105                 110
   agt tat gct agg tta aca gga gtt ttt aga att ctt gaa gtt gga gat         384
   Ser Tyr Ala Arg Leu Thr Gly Val Phe Arg Ile Leu Glu Val Gly Asp
           115                 120                 125
   act tca ggg acc agt cag gat gga act tat ctt gct caa gca aaa ggt      432
   Thr Ser Gly Thr Ser Gln Asp Gly Thr Tyr Leu Ala Gln Ala Lys Gly
       130                 135                 140
   aag cac aca ggg ggc ggt ggt tca cca gat cca gca att tgt ttg tat      480
   Lys His Thr Gly Gly Gly Gly Ser Pro Asp Pro Ala Ile Cys Leu Tyr
   145                 150                 155                 160
   tta gca aaa cct gtt tat ggt aca ggg gaa gat gcc gat aaa caa gtc      528
   Leu Ala Lys Pro Val Tyr Gly Thr Gly Glu Asp Ala Asp Lys Gln Val
                   165                 170                 175
   tcg ttc gat att tat gca gag cgt att tta tat cga ggt gga gaa gga      576
   Ser Phe Asp Ile Tyr Ala Glu Arg Ile Leu Tyr Arg Gly Gly Glu Gly
               180                 185                 190
   gat gga aga gaa att gtt ttc tta aag aat gta aag aag gat gaa gaa      624
   Asp Gly Arg Glu Ile Val Phe Leu Lys Asn Val Lys Lys Asp Glu Glu
           195                 200                 205
   act aat ttt gag tta gaa gtt ggt ttc aaa gaa gat cct aac gat gtc      672
   Thr Asn Phe Glu Leu Glu Val Gly Phe Lys Glu Asp Pro Asn Asp Val
       210                 215                 220
   tct aaa aaa ata cag tat tgt aat gct gtc ata gga gga gat act ttt      720
   Ser Lys Lys Ile Gln Tyr Cys Asn Ala Val Ile Gly Gly Asp Thr Phe
   225                 230                 235                 240
   aat tgg aat att cca gaa cca gaa aga gga aca gaa tcg ggt att aga      768
   Asn Trp Asn Ile Pro Glu Pro Glu Arg Gly Thr Glu Ser Gly Ile Arg
                   245                 250                 255
   tat gga gca tat aga gtt aaa gga gga cga gct caa ata agg tgg gcc      816
   Tyr Gly Ala Tyr Arg Val Lys Gly Gly Arg Ala Gln Ile Arg Trp Ala
               260                 265                 270
   aat acc acc tac caa aag gta gaa aac gta gag gtt aca aat cca gga      864
   Asn Thr Thr Tyr Gln Lys Val Glu Asn Val Glu Val Thr Asn Pro Gly
           275                 280                 285
   cca ata gga gat gtt tat aaa tta aaa aat gtt gct aca ggt cag tat      912
   Pro Ile Gly Asp Val Tyr Lys Leu Lys Asn Val Ala Thr Gly Gln Tyr
       290                 295                 300
   tta tca gat tcg gga gtt agt gct tca gca gta att atg agt gat tca      960
   Leu Ser Asp Ser Gly Val Ser Ala Ser Ala Val Ile Met Ser Asp Ser
   305                 310                 315                 320
   ggt gaa gcg caa aat aat tat tgg act ttt gta gaa agc ggg agt ttg     1008
   Gly Glu Ala Gln Asn Asn Tyr Trp Thr Phe Val Glu Ser Gly Ser Leu
                   325                 330                 335
   ttt aac ata gat aat gaa act ttt ggc ata ctg cgt gca cca ggt gca     1056
   Phe Asn Ile Asp Asn Glu Thr Phe Gly Ile Leu Arg Ala Pro Gly Ala
               340                 345                 350
   ggt ggt cca gga ggt gct tat gtg gta gta agt aca aca aaa gaa ggt     1104
   Gly Gly Pro Gly Gly Ala Tyr Val Val Val Ser Thr Thr Lys Glu Gly
           355                 360                 365
   cct tct tct gat ggc gat aag gtt tgg acg ata cat tat aat gaa tct     1152
   Pro Ser Ser Asp Gly Asp Lys Val Trp Thr Ile His Tyr Asn Glu Ser
       370                 375                 380
   aac gat aca tat cga ttt gaa tcg ggt agt agt ggc aga ttt atg tat        1200
   Asn Asp Thr Tyr Arg Phe Glu Ser Gly Ser Ser Gly Arg Phe Met Tyr
   385                 390                 395                 400
   caa gaa ata aat gga aat gtt act cat atc tcc gct atg aat acc gat        1248
   Gln Glu Ile Asn Gly Asn Val Thr His Ile Ser Ala Met Asn Thr Asp
                   405                 410                 415
   gat aga agt gtt tgg aaa gct att gct gtt gag tct ctg agt gta gat        1296
   Asp Arg Ser Val Trp Lys Ala Ile Ala Val Glu Ser Leu Ser Val Asp
               420                 425                 430
   gag aac gcg att tta gct tct gat gta cgt gta ttt cct aac cca gca        1344
   Glu Asn Ala Ile Leu Ala Ser Asp Val Arg Val Phe Pro Asn Pro Ala
           435                 440                 445
   agc gat agt ttt acg att tcc tta aaa aca ata aat cat gtt aca gtt        1392
   Ser Asp Ser Phe Thr Ile Ser Leu Lys Thr Ile Asn His Val Thr Val
       450                 455                 460
   aat ata tac gat gtt tta gga aat act att ttt aaa agt gaa ttt aat        1440
   Asn Ile Tyr Asp Val Leu Gly Asn Thr Ile Phe Lys Ser Glu Phe Asn
   465                 470                 475                 480
   ggg gat act ata caa ata aga aat aaa ggg caa ttc aaa gct ggg gtt        1488
   Gly Asp Thr Ile Gln Ile Arg Asn Lys Gly Gln Phe Lys Ala Gly Val
                   485                 490                 495
   tat ctt ata cag tta act gat aaa aat aac aac aag tat cat aag aaa        1536
   Tyr Leu Ile Gln Leu Thr Asp Lys Asn Asn Asn Lys Tyr His Lys Lys
               500                 505                 510
   ttg ata gta aaa                                                        1548
   Leu Ile Val Lys
           515
<210> 2
   <211> 516
   <212> PRT
   <213> Formosa agariphila
<400> 2
   Met Leu Glu Lys Thr Thr Leu Lys Asn Ile Ile Leu Ile His Phe Leu
   1               5                   10                  15
   Met Phe Leu Ala Val Val Thr Ala Gln Thr Ala Pro Asp Glu Asp Thr
               20                  25                  30
   Ser Ala Ile Thr Arg Cys Thr Ala Glu Gly Thr Asn Pro Val Arg Glu
           35                  40                  45
   Thr Asp Ile Pro Asn Pro Val Asn Val Gly Thr Ile Asp Asp Arg Ser
       50                  55                  60
   Cys Tyr Ala Asn Tyr Lys Glu Ser Thr Val Tyr Gly Lys Thr Trp Gly
   65                  70                  75                  80
   Val Tyr Asn Ile Thr Phe Asp Ser Asn Asp Phe Asp Thr Ser Leu Gln
                   85                  90                  95
   Pro Arg Ile Glu Arg Ser Leu Ser Arg Ser Ser Glu Thr Gly Ile Gly
               100                 105                 110
   Ser Tyr Ala Arg Leu Thr Gly Val Phe Arg Ile Leu Glu Val Gly Asp
           115                 120                 125
   Thr Ser Gly Thr Ser Gln Asp Gly Thr Tyr Leu Ala Gln Ala Lys Gly
       130                 135                 140
   Lys His Thr Gly Gly Gly Gly Ser Pro Asp Pro Ala Ile Cys Leu Tyr
   145                 150                 155                 160
   Leu Ala Lys Pro Val Tyr Gly Thr Gly Glu Asp Ala Asp Lys Gln Val
                   165                 170                 175
   Ser Phe Asp Ile Tyr Ala Glu Arg Ile Leu Tyr Arg Gly Gly Glu Gly
               180                 185                 190
   Asp Gly Arg Glu Ile Val Phe Leu Lys Asn Val Lys Lys Asp Glu Glu
           195                 200                 205
   Thr Asn Phe Glu Leu Glu Val Gly Phe Lys Glu Asp Pro Asn Asp Val
       210                 215                 220
   Ser Lys Lys Ile Gln Tyr Cys Asn Ala Val Ile Gly Gly Asp Thr Phe
   225                 230                 235                 240
   Asn Trp Asn Ile Pro Glu Pro Glu Arg Gly Thr Glu Ser Gly Ile Arg
                   245                 250                 255
   Tyr Gly Ala Tyr Arg Val Lys Gly Gly Arg Ala Gln Ile Arg Trp Ala
               260                 265                 270
   Asn Thr Thr Tyr Gln Lys Val Glu Asn Val Glu Val Thr Asn Pro Gly
           275                 280                 285
   Pro Ile Gly Asp Val Tyr Lys Leu Lys Asn Val Ala Thr Gly Gln Tyr
       290                 295                 300
   Leu Ser Asp Ser Gly Val Ser Ala Ser Ala Val Ile Met Ser Asp Ser
   305                 310                 315                 320
   Gly Glu Ala Gln Asn Asn Tyr Trp Thr Phe Val Glu Ser Gly Ser Leu
                   325                 330                 335
   Phe Asn Ile Asp Asn Glu Thr Phe Gly Ile Leu Arg Ala Pro Gly Ala
               340                 345                 350
   Gly Gly Pro Gly Gly Ala Tyr Val Val Val Ser Thr Thr Lys Glu Gly
           355                 360                 365
   Pro Ser Ser Asp Gly Asp Lys Val Trp Thr Ile His Tyr Asn Glu Ser
       370                 375                 380
   Asn Asp Thr Tyr Arg Phe Glu Ser Gly Ser Ser Gly Arg Phe Met Tyr
   385                 390                 395                 400
   Gln Glu Ile Asn Gly Asn Val Thr His Ile Ser Ala Met Asn Thr Asp
                   405                 410                 415
   Asp Arg Ser Val Trp Lys Ala Ile Ala Val Glu Ser Leu Ser Val Asp
               420                 425                 430
   Glu Asn Ala Ile Leu Ala Ser Asp Val Arg Val Phe Pro Asn Pro Ala
           435                 440                 445
   Ser Asp Ser Phe Thr Ile Ser Leu Lys Thr Ile Asn His Val Thr Val
       450                 455                 460
   Asn Ile Tyr Asp Val Leu Gly Asn Thr Ile Phe Lys Ser Glu Phe Asn
   465                 470                 475                 480
   Gly Asp Thr Ile Gln Ile Arg Asn Lys Gly Gln Phe Lys Ala Gly Val
                   485                 490                 495
   Tyr Leu Ile Gln Leu Thr Asp Lys Asn Asn Asn Lys Tyr His Lys Lys
               500                 505                 510
   Leu Ile Val Lys
           515
<210> 3
   <211> 783
   <212> DNA
   <213> Formosa agariphila
<220>
   <221> CDS
   <222> (1)..(783)
<400> 3
   caa acc gcg ccc gat gaa gat aca tct gct att aca aga tgt act gct        48
   Gln Thr Ala Pro Asp Glu Asp Thr Ser Ala Ile Thr Arg Cys Thr Ala
   1               5                   10                  15
   gaa ggt aca aac cct gta aga gaa aca gat att cca aac ccc gta aat        96
   Glu Gly Thr Asn Pro Val Arg Glu Thr Asp Ile Pro Asn Pro Val Asn
               20                  25                  30
   gtg gga act atc gat gac cgt agt tgt tat gct aat tat aag gag agt       144
   Val Gly Thr Ile Asp Asp Arg Ser Cys Tyr Ala Asn Tyr Lys Glu Ser
           35                  40                  45
   aca gtt tat gga aaa act tgg ggt gtc tat aac ata acg ttt gat tct       192
   Thr Val Tyr Gly Lys Thr Trp Gly Val Tyr Asn Ile Thr Phe Asp Ser
       50                  55                  60
   aat gat ttt gat acc tct tta caa cca aga ata gaa cgt tca tta agc       240
   Asn Asp Phe Asp Thr Ser Leu Gln Pro Arg Ile Glu Arg Ser Leu Ser
   65                  70                  75                  80
   cgc tcc agc gaa acg ggt att gga agt tat gct agg tta aca gga gtt       288
   Arg Ser Ser Glu Thr Gly Ile Gly Ser Tyr Ala Arg Leu Thr Gly Val
                   85                  90                  95
   ttt aga att ctt gaa gtt gga gat act tca ggg acc agt cag gat gga       336
   Phe Arg Ile Leu Glu Val Gly Asp Thr Ser Gly Thr Ser Gln Asp Gly
               100                 105                 110
   act tat ctt gct caa gca aaa ggt aag cac aca ggg ggc ggt ggt tca       384
   Thr Tyr Leu Ala Gln Ala Lys Gly Lys His Thr Gly Gly Gly Gly Ser
           115                 120                 125
   cca gat cca gca att tgt ttg tat tta gca aaa cct gtt tat ggt aca       432
   Pro Asp Pro Ala Ile Cys Leu Tyr Leu Ala Lys Pro Val Tyr Gly Thr
       130                 135                 140
   ggg gaa gat gcc gat aaa caa gtc tcg ttc gat att tat gca gag cgt       480
   Gly Glu Asp Ala Asp Lys Gln Val Ser Phe Asp Ile Tyr Ala Glu Arg
   145                 150                 155                 160
   att tta tat cga ggt gga gaa gga gat gga aga gaa att gtt ttc tta       528
   Ile Leu Tyr Arg Gly Gly Glu Gly Asp Gly Arg Glu Ile Val Phe Leu
                   165                 170                 175
   aag aat gta aag aag gat gaa gaa act aat ttt gag tta gaa gtt ggt       576
   Lys Asn Val Lys Lys Asp Glu Glu Thr Asn Phe Glu Leu Glu Val Gly
               180                 185                 190
   ttc aaa gaa gat cct aac gat gtc tct aaa aaa ata cag tat tgt aat       624
   Phe Lys Glu Asp Pro Asn Asp Val Ser Lys Lys Ile Gln Tyr Cys Asn
           195                 200                 205
   gct gtc ata gga gga gat act ttt aat tgg aat att cca gaa cca gaa       672
   Ala Val Ile Gly Gly Asp Thr Phe Asn Trp Asn Ile Pro Glu Pro Glu
       210                 215                 220
   aga gga aca gaa tcg ggt att aga tat gga gca tat aga gtt aaa gga       720
   Arg Gly Thr Glu Ser Gly Ile Arg Tyr Gly Ala Tyr Arg Val Lys Gly
   225                 230                 235                 240
   gga cga gct caa ata agg tgg gcc aat acc acc tac caa aag gta gaa       768
   Gly Arg Ala Gln Ile Arg Trp Ala Asn Thr Thr Tyr Gln Lys Val Glu
                       245                 250                 255
   aac gta gag gtt aca                                                        783
   Asn Val Glu Val Thr
               260
<210> 4
   <211> 261
   <212> PRT
   <213> Formosa agariphila
<400> 4
   Gln Thr Ala Pro Asp Glu Asp Thr Ser Ala Ile Thr Arg Cys Thr Ala
   1               5                   10                  15
   Glu Gly Thr Asn Pro Val Arg Glu Thr Asp Ile Pro Asn Pro Val Asn
               20                  25                  30
   Val Gly Thr Ile Asp Asp Arg Ser Cys Tyr Ala Asn Tyr Lys Glu Ser
           35                  40                  45
   Thr Val Tyr Gly Lys Thr Trp Gly Val Tyr Asn Ile Thr Phe Asp Ser
       50                  55                  60
   Asn Asp Phe Asp Thr Ser Leu Gln Pro Arg Ile Glu Arg Ser Leu Ser
   65                  70                  75                  80
   Arg Ser Ser Glu Thr Gly Ile Gly Ser Tyr Ala Arg Leu Thr Gly Val
                   85                  90                  95
   Phe Arg Ile Leu Glu Val Gly Asp Thr Ser Gly Thr Ser Gln Asp Gly
               100                 105                 110
   Thr Tyr Leu Ala Gln Ala Lys Gly Lys His Thr Gly Gly Gly Gly Ser
           115                 120                 125
   Pro Asp Pro Ala Ile Cys Leu Tyr Leu Ala Lys Pro Val Tyr Gly Thr
       130                 135                 140
   Gly Glu Asp Ala Asp Lys Gln Val Ser Phe Asp Ile Tyr Ala Glu Arg
   145                 150                 155                 160
   Ile Leu Tyr Arg Gly Gly Glu Gly Asp Gly Arg Glu Ile Val Phe Leu
                   165                 170                 175
   Lys Asn Val Lys Lys Asp Glu Glu Thr Asn Phe Glu Leu Glu Val Gly
               180                 185                 190
   Phe Lys Glu Asp Pro Asn Asp Val Ser Lys Lys Ile Gln Tyr Cys Asn
               195                 200                 205
   Ala Val Ile Gly Gly Asp Thr Phe Asn Trp Asn Ile Pro Glu Pro Glu
       210                 215                 220
   Arg Gly Thr Glu Ser Gly Ile Arg Tyr Gly Ala Tyr Arg Val Lys Gly
   225                 230                 235                 240
   Gly Arg Ala Gln Ile Arg Trp Ala Asn Thr Thr Tyr Gln Lys Val Glu
                   245                 250                 255
   Asn Val Glu Val Thr
               260
<210> 5
   <211> 273
   <212> PRT
   <213> Formosa agariphila
<220>
   <221> misc_feature
   <222> (1)..(273)
   <223> séquence protéique du module catalytique de l'ulvane lyase de Formosa agariphila DSM 15362 produite sous forme recombinante
<400> 5
   Met Gly Ser Ser His His His His His His Gly Ser Gln Thr Ala Pro
   1               5                   10                  15
   Asp Glu Asp Thr Ser Ala Ile Thr Arg Cys Thr Ala Glu Gly Thr Asn
               20                  25                  30
   Pro Val Arg Glu Thr Asp Ile Pro Asn Pro Val Asn Val Gly Thr Ile
           35                  40                  45
   Asp Asp Arg Ser Cys Tyr Ala Asn Tyr Lys Glu Ser Thr Val Tyr Gly
       50                  55                  60
   Lys Thr Trp Gly Val Tyr Asn Ile Thr Phe Asp Ser Asn Asp Phe Asp
   65                  70                  75                  80
   Thr Ser Leu Gln Pro Arg Ile Glu Arg Ser Leu Ser Arg Ser Ser Glu
                   85                  90                  95
   Thr Gly Ile Gly Ser Tyr Ala Arg Leu Thr Gly Val Phe Arg Ile Leu
               100                 105                 110
   Glu Val Gly Asp Thr Ser Gly Thr Ser Gln Asp Gly Thr Tyr Leu Ala
           115                 120                 125
   Gln Ala Lys Gly Lys His Thr Gly Gly Gly Gly Ser Pro Asp Pro Ala
       130                 135                 140
   Ile Cys Leu Tyr Leu Ala Lys Pro Val Tyr Gly Thr Gly Glu Asp Ala
   145                 150                 155                 160
   Asp Lys Gln Val Ser Phe Asp Ile Tyr Ala Glu Arg Ile Leu Tyr Arg
                   165                 170                 175
   Gly Gly Glu Gly Asp Gly Arg Glu Ile Val Phe Leu Lys Asn Val Lys
               180                 185                 190
   Lys Asp Glu Glu Thr Asn Phe Glu Leu Glu Val Gly Phe Lys Glu Asp
           195                 200                 205
   Pro Asn Asp Val Ser Lys Lys Ile Gln Tyr Cys Asn Ala Val Ile Gly
       210                 215                 220
   Gly Asp Thr Phe Asn Trp Asn Ile Pro Glu Pro Glu Arg Gly Thr Glu
   225                 230                 235                 240
   Ser Gly Ile Arg Tyr Gly Ala Tyr Arg Val Lys Gly Gly Arg Ala Gln
                   245                 250                 255
   Ile Arg Trp Ala Asn Thr Thr Tyr Gln Lys Val Glu Asn Val Glu Val
               260                 265                 270
   Thr
<210> 6
   <211> 47
   <212> PRT
   <213> Artificial Sequence
<220>
   <223> Séquence signal
<400> 6
   Met Val Phe Phe Lys Asp Leu Phe Ile Phe Lys Ser Leu Ile Lys Gly
   1               5                   10                  15
   Ser Leu Tyr Ser Gly His Met Lys Lys Lys Leu Leu Asn Tyr Leu Pro
               20                  25                  30
   Leu Phe Ala Leu Met Leu Phe Thr Val Ser Met Met Ala Gln Thr
           35                  40                  45

## Description

### Domaine de l'invention

La présente invention se rapporte à une nouvelle ulvane lyase, à une séquence d'acide nucléique codant pour cette enzyme, à un vecteur comprenant ladite séquence codante, à un procédé de fabrication de cette ulvane lyase, ainsi qu'à un procédé de production de d'oligosaccharides d'ulvanes à activité biologique utilisant cette enzyme.

La présente invention trouve ses applications principalement dans l'agriculture, les industries de la nutrition animale et humaine, de la cosmétique, de la pharmacie, etc... mais également dans le domaine de la recherche en laboratoire pour la production de molécules standard, marqueurs de taille, etc... ou pour l'analyse fine de la structure et des compositions de polysaccharides d'ulvanes de nature et provenance variées.

Dans la description ci-dessous, les références entre crochets ([ ]) renvoient à la liste des références présentées à la fin du texte.

### Etats de la technique

Les macroalgues vertes de la famille des Ulvaceae sont présentes partout sur le globe terrestre et sont très communément rencontrées sur les côtes. Ces algues sont fréquemment impliquées dans la prolifération algale favorisée par l'eutrophisation des eaux côtières donnant lieu aux « marées vertes ». Toutefois jusqu'à présent, elles constituent une énorme biomasse sous-exploitée en biotechnologie, et utilisée essentiellement comme compost.

Les polysaccharides anioniques complexes majeurs présents dans la paroi des ulves, les ulvanes, sont des polysaccharides uroniques et sulfatés possédant des structures originales et représentent une source de biopolymères dont les fonctionnalités sont encore peu explorées.

Les ulvanes et les oligosaccharides dérivés ont de nombreuses propriétés biologiques intéressantes, notamment due à leur ressemblance aux glycosaminoglycanes sulfatés des animaux (e.g. héparines, dermatanes sulfates, etc...). Les ulvanes sont composés de différentes unités de répétition disaccharidiques construites avec des unités de L-rhamnoses, acides D-glucuroniques, acides L-iduroniques, D-xyloses et de groupement ester-sulfates. Les deux principaux motifs de répétition sont appelés acide aldobiuronique, ou acides ulvanobiuroniques, ou respectivement A (A_{3S}) et B (B_{3S}), dont les formules sont les suivantes :

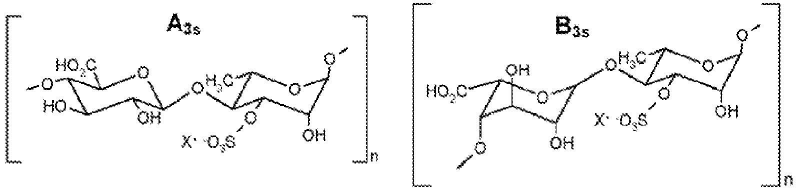

Le motif A (A_{3S}) est l'acide beta-D-1,4-glucuronique (1→4) alpha-L-1,4-rhamnose 3-sulfate. Le motif B (B3S) est l'acide alpha-L-1,4-iduronique (1→4) alpha-L-1,4-rhamnose 3-sulfate. Il est noté que l'acide L-iduronique est le C5-epimère de l'acide D-glucuronique. Les acides uroniques sont parfois remplacés par des résidus xylose sulfatés en O-2.

Les ulvanes possèdent des propriétés physicochimiques uniques qui en font des candidats attractifs pour de nouvelles applications agroalimentaires, pharmaceutiques, et cosmétiques. Les ulvanes possèdent des structures très originales composées de sucres ou monosaccharides rares comme le rhamnose et l'acide iduronique. Le rhamnose est un composé important des antigènes de surface de nombreux microorganismes reconnus spécifiquement par les lectines de mammifères. Il est également utilisé pour la synthèse d'arômes. L'acide iduronique est utilisé pour la synthèse des glycosaminoglycanes, par exemple l'héparine. Ainsi les ulvanes peuvent être considérés comme des biomimétiques des glycosaminoglycanes des animaux et de l'homme, en raison de la présence conjointe de sucres sulfatés et d'acides D-glucuroniques et L-iduronique.

En plus des monomères, les ulvanes et oligo-ulvanes présentent des propriétés biologiques intéressantes. En effet, des travaux ont montré, par exemple, que les oligo-ulvanes ont des activités antitumorales, antivirales, notamment antigrippales, et anticoagulantes. Une liste non-exhaustive d'applications potentielles des ulvanes a été proposée par Lahaye et Robic (Biomacromolecules, 8 : 1765-1774, 2007) [1].

Dans ce contexte une meilleure compréhension de la structure des ulvanes et le développement de procédés permettant de fragmenter les ulvanes sous forme oligomérique ou monomérique revêt un très grand intérêt.

Actuellement faute de moyens permettant de mieux connaitre les ulvanes et de les dégrader plus efficacement, les algues, notamment les algues vertes, sont essentiellement compostées et peu de valorisation industrielle n'en est faite. Ceci est d'autant plus déplorable que la source est abondante et parfois gênante en termes de pollution de nos côtes maritimes. Leur élimination est actuellement réalisée par voie de compost.

L'hydrolyse acide est essentiellement la seule méthode utilisée pour libérer des oligosaccharides d'ulvanes, mais cette méthode est aléatoire et altère la structure du polymère.

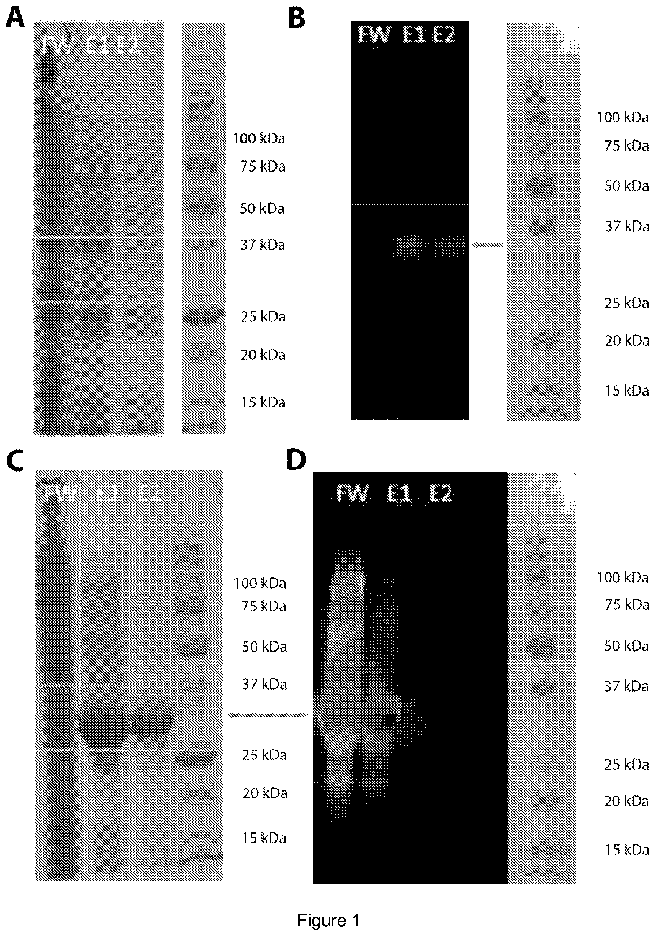

Certaines bactéries marines sécrètent des enzymes impliquées dans la dégradation spécifique des polysaccharides algaux. L'utilisation d'enzyme spécifique permet de libérer de façon reproductible des oligosaccharides tout en préservant leur structure et activité biologique. Une ulvane lyase de *Nonlabens ulvanivorans* PLR (précédemment nommé *Persicivirga ulvanivorans* PLR) a été décrite et protégée par brevet (Nyvall Collen et al., J. Biol. Chem., 286(49): 42063-42071, 2011 ; Demande internationale WO 2011157966) [2,3]. Toutefois cette enzyme est extrêmement faiblement exprimée sous une forme soluble et active dans *Escherichia coli* [2] (voir aussi résultat propre ci-dessous, Figure 1); ce qui empêche en pratique toute exploitation industrielle de cette enzyme.

Il existe donc un réel besoin d'un nouveau moyen et/ou procédé palliant les défauts, inconvénients et obstacles de l'art antérieur, en particulier d'un nouveau moyen et/ou procédé permettant de dégrader les ulvanes afin de valoriser cette bio-ressource, issue notamment des algues vertes, et ainsi libérer de façon contrôlée et reproductible de nouveaux oligosaccharides d'ulvanes de structures définies, et ce en préservant la structure fine et donc les propriétés biologiques desdits oligosaccharides dans la perspective d'applications cosmétiques, agroalimentaires et médicales.

### Description de l'invention

Les Inventeurs ont répondu à ce besoin en identifiant, clonant et surexprimant de façon hétérologue dans *Escherichia coli* et purifiant, pour la première fois, une nouvelle ulvane lyase active, à partir du génome complet de la bactérie marine *Formosa agariphila* DSM 15362 (Mann et al., Appl. Env. Microbiol., 79: 6813-6822, 2013; Nedashkovskaya et al., Int. J. Syst. Evol. Microbiol., 56 : 161-167, 2006) [4, 5]. Partant de [4], une protéine ulvanlyase de 500 acides aminés a été décrite (DATABASE UniProt no. T2KNC2 ; DATABASE ENA no. CDF79931) [11, 12]. Par ailleurs, l'utilisation de cette enzyme pour la dégradation complète de l'ulvane a permis d'isoler et de purifier de nouveaux oligosaccharides d'ulvanes (ou oligo-ulvanes) ayant une structure chimique unique, sulfatée. Ces oligosaccharides sulfatés sont potentiellement intéressants pour des applications en biomédical, cosmétique, nutrition, élicitation de plantes, etc... Cette enzyme permet également d'augmenter le rendement d'extraction d'autres composés (e.g. protéines, pigments, métabolites secondaires) à partir des macroalgues vertes contenant de l'ulvane. Ainsi les Inventeurs ont mis en évidence que l'ulvane lyase de *Formosa agariphila* DSM 15 362 permet de : (i) de dépolymariser l'ulvane (polysaccharide majeur des macroalgues vertes), libérant des oligosaccharides à activité biologique en préservant leur structure native ; (ii) dégrader directement des macroalgues vertes pour générer des fractions contenant des oligosaccharides à activité biologique ; et (iii) augmenter le rendement d'extraction d'autres composés (e.g. protéines, pigments, etc...) à partir de macroalgues vertes.

La présente invention se rapporte donc à une ulvane lyase de *Formosa agariphila* DSM 15362 comprenant la séquence en acides aminés SEQ ID NO : 4 ou 5, et comprenant en outre à son extrémité N-terminale une séquence signal de séquence SEQ ID NO : 6. Les séquences SEQ ID NOs : 4 et 5 représentent, respectivement, le module catalytique de l'ulvane lyase de *Formosa agariphila* DSM 15362 et ledit module produit sous forme recombinante (dans le cytoplasme d'E. *coli.* La séquence additionnelle en N-terminal est une étiquette poly-histidine pour faciliter la purification). Cette séquence signal peut être clivée après synthèse de la protéine ou non. Les procédés de clivage connus de l'Homme du métier peuvent être utilisés, par exemple ceux impliquant des protéases spécifiques d'un site de clivage. On choisit alors une séquence signal présentant un tel site.

Toutes ces séquences en acides aminés n'ont jamais été décrites dans l'art antérieur comme étant actives pour dégrader les ulvanes par dépolymérisation selon la présente invention. La présente invention se rapporte donc également à l'utilisation d'une séquence en acides aminés telle que définie ci-dessus pour la dégradation spécifique et complète de l'ulvane pour produire des oligosaccharides d'ulvane (ou oligo-ulvanes).

La présente invention se rapporte également à un acide nucléique codant pour une ulvane lyase selon l'invention. Il peut s'agir par exemple d'un acide nucléique comprenant la séquence SEQ ID NO : 3.

La présente invention se rapporte également à un vecteur comprenant un acide nucléique selon l'invention. Le vecteur peut-être un des vecteurs connus de l'Homme du métier pour fabriquer des protéines par recombinaison génétique, par exemple un vecteur d'expression. Il est choisi en général notamment en fonction de l'hôte cellulaire choisi.

La présente invention se rapporte également à une cellule hôte comprenant une séquence d'acide nucléique selon l'invention, ou un vecteur selon l'invention. La cellule hôte ou hôte cellulaire peut être tout hôte approprié pour la fabrication de l'ulvane lyase de la présente invention à partir de l'acide nucléique ou du vecteur de l'invention. Il peut s'agir par exemple d'*E*. *coli*, de *Pischia pastoris*, de *Saccharomyces cerevisiae*, de cellules d'insectes, par exemple un système cellules d'insectes-baculovirus (par exemple cellules d'insecte SF9 utilisant un système d'expression de baculovirus), de mammifères.

La présente invention se rapporte également à un procédé de fabrication d'une ulvane lyase selon l'invention par recombinaison génétique utilisant un acide nucléique selon l'invention, ou un vecteur selon l'invention. Les procédés de recombinaison génétique connus de l'Homme du métier sont utilisables. L'origine marine ou terrestre n'influence pas la possibilité de recombinaison et d'expression hétérologue. L'ulvane lyase de la présente invention peut également être produite par la mise en culture du microorganisme *F. agariphila* dans un milieu permettant la croissance dudit microorganisme marin. Il peut s'agir par exemple d'un milieu de culture liquide ZoBell. Le pH de culture est de préférence compris entre 7,5 et 8,4, de préférence à pH 8,0. La température de culture est de préférence comprise entre 4 et 33°C, de préférence à 22°C. La culture est de préférence réalisée avec une concentration de NaCl de 10 à 80 g/l, de préférence de 35 g/l [5]. Ces procédés de fabrication de l'ulvane lyase de l'invention peuvent comprendre en outre une étape de récupération de l'ulvane lyase. Cette étape de récupération ou d'isolement peut être réalisée par tout moyen connu de l'Homme du métier. Il peut s'agir par exemple d'une technique choisie parmi une électrophorèse, un tamisage moléculaire, une ultracentrifugation, une précipitation différentielle, par exemple au sulfate d'ammonium, par ultrafiltration, une filtration sur membrane ou sur gel, un échange d'ions, une élution sur hydroxyapatite, une séparation par interactions hydrophobes, ou tout autre moyen connu.

Le microorganisme *F. agariphila* ou toute cellule hôte transformée pour une fabrication par recombinaison génétique selon l'invention, peut également être utilisé directement pour dégrader des ulvanes, dans leur milieu naturel ou en culture. Les conditions permettant la dégradation des ulvanes, lorsqu'on utilise une cellule hôte ou microorganisme sont, en culture, un système discontinu ou continu, par exemple un réacteur de culture contenant un milieu de culture approprié au développement du microorganisme.

La présente invention se rapporte également à un procédé de dégradation d'ulvanes comprenant une étape de mise en contact des ulvanes avec une ulvane lyase selon l'invention, ou avec une cellule hôte selon l'invention, dans des conditions permettant la dégradation des ulvanes par digestion enzymatique par ladite ulvane lyase ou ladite cellule hôte ou ledit microorganisme *F. agariphila.*

Pour la digestion enzymatique, la détermination des constantes de Michaelis Menten (Km et Vmax) permet aisément à l'Homme du métier de trouver les conditions optimales de concentration de l'ulvane lyase utilisée et de concentration des ulvanes pour la dégradation des ulvanes dans le milieu où elles se trouvent ou dans le milieu dans lequel elles ont été placées. Le pH peut être également compris de préférence entre 6.0 et 8.5, de préférence à 7.5. Il s'agit en effet de la gamme de pH optimale. La température (optimale) est de préférence comprise entre 30 et 40°C. La force ionique peut être comprise de préférence entre 0 et 300 mM NaCl, de préférence à 30 mM NaCl.

La présente invention permet avantageusement de mobiliser la très grande ressource d'algues actuellement inexploitée, notamment d'algues vertes. L'invention permet en outre de favoriser la biodégradation des algues, notamment des algues vertes, de produire des molécules originales, qui sont des fragments d'ulvanes ou oligo-ulvanes, par exemple des oligosaccharides, par exemple aussi des hydrocolloïdes, et d'offrir une source nouvelle de monosaccharides rares pour des applications cosmétiques, agroalimentaires et médicaments ou formulations pharmaceutiques et parapharmaceutiques.

Les produits de dégradation des ulvanes donnent accès à de nouveaux produits qui peuvent être des actifs alimentaires, cosmétiques, pharmaceutiques et parapharmaceutiques utilisables dans les domaines agroalimentaires, cosmétiques, pharmaceutiques et parapharmaceutiques. Ces nouveaux produits peuvent également être des produits non actifs mais présentant une neutralité et/ou stabilité qui est très intéressante pour une utilisation dans chacun de ces domaines.

L'utilisation de l'ulvane lyase de l'invention donne en outre accès à des monosaccharides rares utilisables comme synthons en glycochimie. La dégradation des ulvanes par l'ulvane lyase de la présente invention combinée à d'autres enzymes peut donner accès à l'acide iduronique (sucre rare) utilisé pour la synthèse des glycosaminoglycans de synthèse.

La présente invention ouvre également de nouvelles perspectives d'utilisation de ces algues pour des applications en bioénergie et en chimie. La production de fragments oligosaccharides peut donner de molécules de base pour la fabrication d'autres molécules. La dépolymarisation de l'ulvane devrait faciliter la fermentation par des microorganismes conduisant à la production de méthane par exemple.

D'autres caractéristiques et avantages apparaitront encore à l'Homme du métier à la lecture des exemples ci-dessous, donnés à titre illustratif et non limitatif, en référence aux figures annexées.

### Brève description des figures

- La figure 1 représente la comparaison de l'expression dans *E. coli* BI2(DE3) du module catalytique de l'ulvane lyase de *N. ulvanivorans PLR* (A et B) et *F. agariphila* DSM 15362 (C et D). Les panneaux A et C correspondent à des analyses SDS-PAGE. Les panneaux B et D correspondent à des analyses western-blot avec un anticorps anti-His-Tag. FW= Flow-through de la chromatographie d'affinité au nickel ; E1 et E2 : éluats 1 et 2 de la chromatographie d'affinité au nickel.
- La figure 2 représente un test d'activité de l'ulvane lyase recombinante BN863_22190_cat en fonction de la concentration en NaCl (A), de la nature du tampon (B) et du pH (pour le meilleur tampon, C). L'activité a été suivie par spectrophotométrie à 235 nm.
- La figure 3 représente une analyse FACE (Fluorophore-assisted Carbohydrate Electrophoresis) des oligo-ulvanes libérés par une digestion extensive d'ulvane par l'ulvane lyase recombinante BN863_22190_cat. Ces échantillons sont nommés FracX où X est un numéro de fraction correspondant à la purification de ces oligosaccharides par chromatographie d'exclusion de taille. Les oligosaccharides DP2 et DP4 sont des oligo-ulvanes standards libérés par l'ulvane lyase sauvage de *Nonlabens ulvanivorans* PLR [1].
- La figure 4 représente un spectre de masse de la fraction 48 enregistré sur la plateforme BIBS (INRA, Nantes). Les pics principaux de masse / charge (m/z) correspondent à un disaccharide Δ-R3S.
- La figure 5 représente un spectre de masse de la fraction 39 enregistré sur la plateforme BIBS (INRA, Nantes). Les pics principaux de masse / charge (m/z) correspondent à un tetrasaccharide Δ-R3S-Xyl-R3S.

### EXEMPLES

### Exemple 1 : Expression de l'ulvane lyase de Formosa agariphila

Le gène codant pour cette ulvane lyase (BN863_22190, SEQ ID NO : 1) a été identifié dans le génome de *F. agariphila* DSM 15362 par homologie de séquence avec l'ulvane lyse de *Nonlabens ulvanivorans* PLR (anciennement *Persicivirga ulvanivorans*) [2, 3].

La protéine codée par ce gène a une masse moléculaire de 56630 Da (516 résidus, SEQ ID NO : 2). Elle possède une architecture modulaire avec un peptide signal en N-terminal suivie d'un module catalytique, d'un module de fonction inconnue et d'un module de sécrétion de type IX en C-terminal.

Sur la totalité de sa séquence, cette protéine possède 52% d'identité de séquence avec celle de l'ulvane lyase de *N. ulvanivorans* PLR, comme déterminé par alignement de séquence par le logiciel CLUSTALW. Le module catalytique de l'ulvane lyase de *F. agariphila* DSM 15362 (résidus 25-285, 28738 Da) présente 66% d'identité de séquence avec la séquence du module catalytique de l'ulvane lyase de *N. ulvanivorans* (résidus 24-291).

La séquence nucléotidique correspondant au module catalytique de l'ulvane lyase de *F. agariphila* DSM 15362 a été clonée dans le vecteur pFO4 (dérivé du vecteur commercial pET15b, [6]), et la protéine recombinante correspondante a été surexprimée dans *Escherichia coli* (SEQ ID NO : 5). A titre de comparaison, un travail similaire a été effectué pour le module catalytique de l'ulvane lyase de *N. ulvanivorans* PLR.

Les résultats présentés dans la figure 1 montrent que le module catalytique de l'ulvane lyase de *N. ulvanivorans* PLR n'est pas visible par électrophorèse sur gel de polyacrylamide dénaturant coloré au bleu de Coomassie (figure 1A), même après purification par chromatographie d'affinité au nickel. Cette enzyme a été néanmoins faiblement détectée par un anticorps anti-Histidine-TAG (figure 1B, cf. flèche) ; ce qui a confirmé une très faible expression soluble de cette protéine (quelques µg par litre de culture). Par contraste, le module catalytique de l'ulvane lyase de *F. agariphila* DSM 15362 (appelé par la suite BN863_22190_cat) est très fortement exprimé sous forme soluble, comme indiqué par la large bande visible à ∼29 kDa en SDS-PAGE (figure 1C). Ceci a été confirmé en western-blot (figure 1D), qui est une technique beaucoup plus sensible que le SDS-PAGE. Le rendement de production de cette protéine recombinante était de 30 mg par litre de culture, soit au moins 10 000 fois plus que l'ulvane lyase recombinante de *N. ulvanivorans* PLR.

### Exemple 2 : Activité biologique de l'ulvane lyase de Formosa agariphila

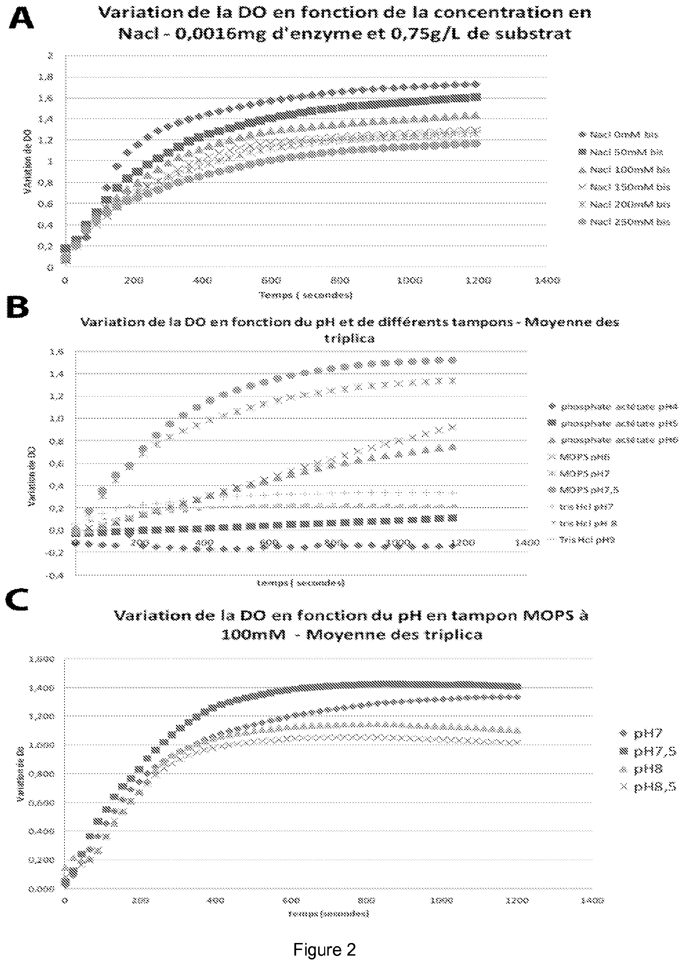

Un test d'activité classiquement utilisé pour les polysaccarides lyases (*e.g.* [7, 8]) a été réalisé pour le BN863_22190_cat. Il consiste en le suivie de l'absorbance à 235 nm du milieu réactionnel. En effet les polysaccharide lyases libèrent des oligosaccharides présentant un monosaccharide insaturée à l'extrémité non-réductrice qui absorbent à 235 nm. Ce test a ainsi permis de déterminer pour l'enzyme la salinité optimale du milieu (Figure 2A), le tampon optimal (Figure 2B) et le pH optimal avec le meilleur tampon (Figure 2C).

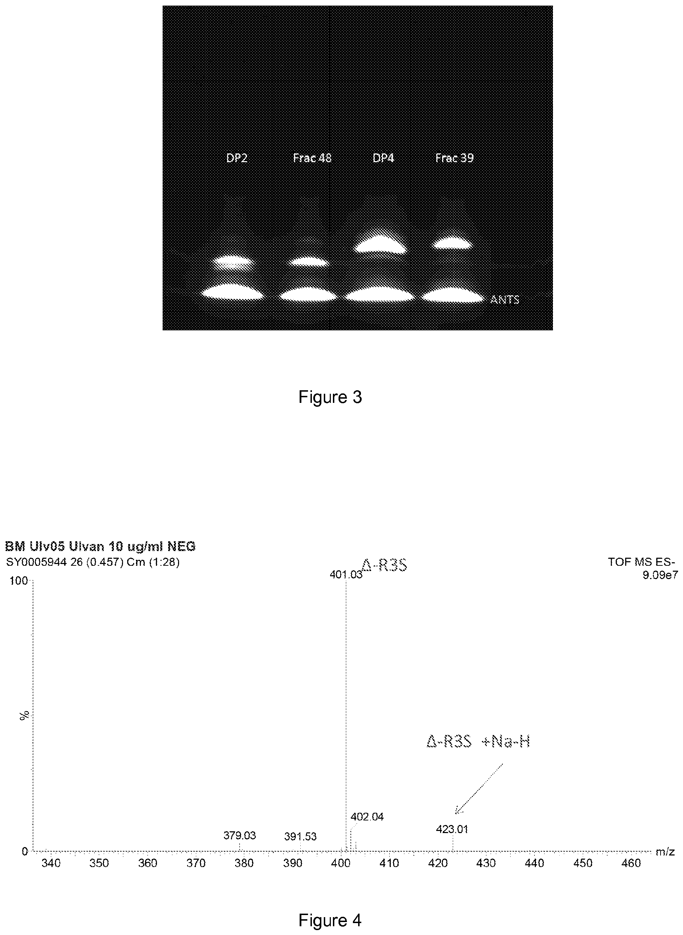

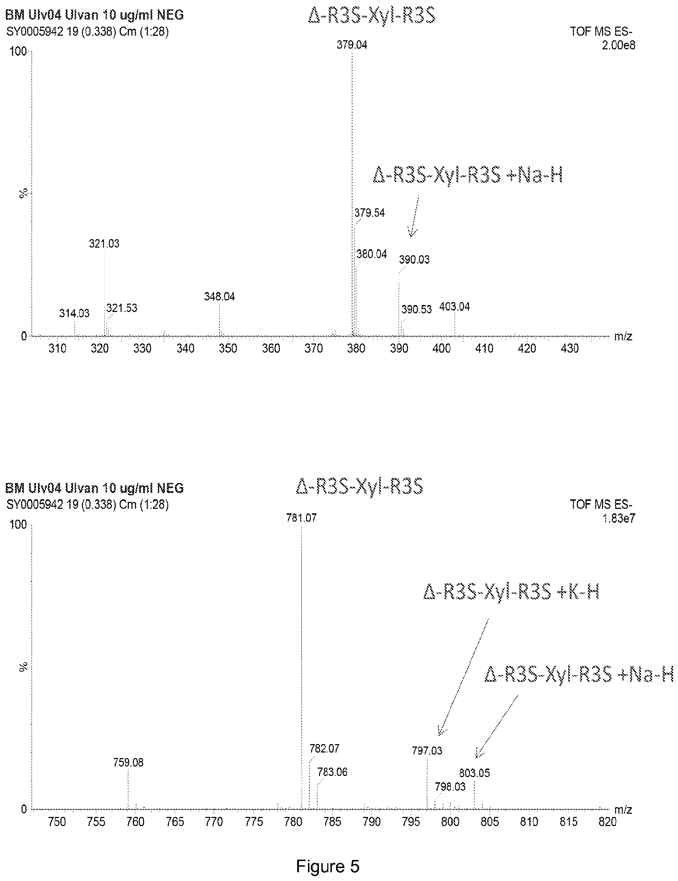

Une dégradation extensive d'un ulvan pur (ULV100, Elicityl, France) a été également entreprise en utilisant BN863_22190_cat. Les oligo-ulvanes libérés ont purifiés par chromatographie d'exclusion de taille sur un système de trois colonnes Superdex 30 interconnectés (GE Healthcare, France). Ces oligosaccharides purifiés ont été ensuite analysés par la technique appelée FACE (Fluorophore-assisted Carbohydrate Electrophoresis). L'extrémité réductrice des oligosaccharides a ainsi été marquée par une molécule fluorescente (8-aminonaphthalene-1,3,6-trisulfonic acid, ANTS) comme précédemment décrit [9]. Les oligosaccharides marqués sont ensuite analysés par électrophorèse en gel de polyacrylamide. Des oligo-ulvanes précédemment obtenus au laboratoire par l'ulvane lyase sauvage de *N. ulvanivorans* PLR [2] ont été également utilisés comme standards (DP2 : le disaccharide Δ-R3S ; DP4 : le tétrasaccharide Δ-R3S-Xyl-R3S ; les structures de DP2 et DP4 ont été déterminées par RMN [2]). La figure 3 montre que les oligosaccharides terminaux « Fraction 48 » et « Fraction 39 » migrent à la même taille que les standards DP2 et DP4, respectivement. La structure de ces oligosaccharides générés par BN863_22190_cat. a été confirmée par spectrométrie de masse (Plateforme BIBS, INRA, Nantes) (Figures 4 et 5). Par conséquent, comme l'ulvane lyase sauvage de *N. ulvanivorans* PLR, BN863_22190_cat. clive la liaison beta-1,4 entre les résidus D-glucuronique acide (GIcA) / L-iduronique acide (IduA) et le rhamnose-3-sulfate (R3S) libérant de manière endolytique des oligosaccharides de tailles différentes présentant un sucre insaturé Δ à l'extrémité non réductrice. Les produits terminaux principaux sont le disaccharide Δ-R3S, et le tétrasaccharide Δ-R3S-Xyl-R3. Les tétrasaccharides Δ-R3S-GlcA-R3S et Δ-R3S-IduA-R3S décrits aussi dans l'article Nyvall Collen et al., JBC, 2011 ne sont pas observé dans l'expérience en raison de la dégradation extensive de l'ulvane par BN863_22190_cat. En effet ces deux types de tétrasaccharides sont clivés en deux par BN863_22190_cat, libérant des disaccharides Δ-R3S (Figure 3). Le tétrasaccharide Δ-R3S-Xyl-R3S est par contre un produit terminal résistant au clivage enzymatique (Figure 3), en raison de la présence d'un résidu xylose en position 3 à partir de l'extrémité non-réductrice.

Les résultats montrent une forte activité de dégradation de l'ulvane par BN863_22190-cat, confirmée par mesure de l'absorbance à 235 nm et par l'analyse électrophorétique et par spectrométrie de masse des oligo-ulvanes purifiés. A titre de comparaison, seule une activité résiduelle a été détectée en suivant la dégradation de l'ulvane par l'ulvane lyase de *N. ulvanivorans* par spectrophotométrie à 240 nm (données non représentées).

### Références

1. Lahaye et Robic, Biomacromolecules, 8 : 1765-1774, 2007
2. Nyvall Collen et al., J. Biol. Chem., 286(49) : 42063-42071, 2011
3. Demande internationale WO 2011/157966
4. Mann et al., Appl. Env. Microbiol., 79 : 6813-6822, 2013
5. Nedashkovskaya, et al (2006). Formosa agariphila sp. nov., a budding bacterium of the family Flavobacteriaceae isolated from marine environments, and emended description of the genus Formosa. Int.J.Syst.Evol.Microbiol. 56 : 161-167.
6. Groisillier et al., Microb Cell Fact. 9:45, 2010
7. Michel et al., J. Biol. Chem., 31 : 32882-32896, 2004
8. Thomas et al., J. Biol. Chem., 32 : 23021-23037, 2013
9. Jackson, Biochem. J., 270 : 705-713, 1990.
10. Petersen et al., Nature Methods, 8 : 785-786, 2011
11. DATABASE UniProt no. T2KNC2
12. DATABASE ENA no. CDF79931 ;

## Revendications

1. Ulvane lyase comprenant la séquence SEQ ID NO : 4 ou 5, et comprenant en outre à son extrémité N-terminale une séquence signal de séquence SEQ ID NO : 6.

2. Acide nucléique codant pour une ulvane lyase telle que définie dans la revendication 1.

3. Acide nucléique selon la revendication 2 comprenant la séquence SEQ ID NO : 3.

4. Vecteur comprenant un acide nucléique selon l'une quelconque des revendications 2 ou 3.

5. Cellule hôte comprenant une séquence d'acide nucléique selon l'une quelconque des revendications 2 ou 3, ou un vecteur selon la revendication 4.

6. Procédé de fabrication d'une ulvane lyase telle que définie dans la revendication 1 par recombinaison génétique utilisant un acide nucléique selon l'une quelconque des revendications 2 ou 3, ou un vecteur selon la revendication 4.

7. Procédé de dégradation d'ulvanes comprenant une étape de mise en contact des ulvanes avec une ulvane lyase selon la revendication 1, ou avec une cellule hôte selon la revendication 5, dans des conditions permettant la dégradation des ulvanes par digestion enzymatique par ladite ulvane lyase ou ladite cellule hôte.

8. Utilisation d'une ulvane lyase telle que définie dans la revendication 1 pour la production d'oligo-ulvanes.

## Patentansprüche

1. Ulvanlyase, umfassend die Sequenz von SEQ ID NO: 4 oder 5 und außerdem umfassend an ihrem N-terminalen Ende eine Signalsequenz der Sequenz von SEQ ID NO: 6.

2. Nukleinsäure, die eine Ulvanlyase nach Anspruch 1 codiert.

3. Nukleinsäure nach Anspruch 2, umfassend die Sequenz von SEQ ID NO: 3.

4. Vektor, umfassend eine Nukleinsäure nach einem der Ansprüche 2 oder 3.

5. Wirtszelle, umfassend eine Nukleinsäuresequenz nach einem der Ansprüche 2 oder 3 oder einen Vektor nach Anspruch 4.

6. Verfahren zur Herstellung einer Ulvanlyase nach Anspruch 1 durch genetische Rekombination unter Verwendung einer Nukleinsäure nach einem der Ansprüche 2 oder 3 oder eines Vektors nach Anspruch 4.

7. Verfahren zum Abbau von Ulvanen, umfassend einen Schritt des Inkontaktbringens von Ulvanen mit einer Ulvanlyase nach Anspruch 1 oder mit einer Wirtszelle nach Anspruch 5 unter Bedingungen, die den Abbau der Ulvane mittels enzymatischem Verdau durch die Ulvanlyase oder die Wirtszelle gestatten.

8. Verwendung einer Ulvanlyase nach Anspruch 1 zur Herstellung von Oligoulvanen.

## Claims

1. Ulvan lyase comprising the sequence SEQ ID NO: 4 or 5, and further comprising at its N-terminal end a signal sequence of sequence SEQ ID NO: 6.

2. Nucleic acid coding for an ulvan lyase as defined in claim 1.

3. Nucleic acid according to claim 2 comprising the sequence SEQ ID NO: 3.

4. Vector comprising a nucleic acid according to either one of claims 2 or 3.

5. Host cell comprising a nucleic acid sequence according to either one of claims 2 or 3, or a vector according to claim 4.

6. Process for production of an ulvan lyase as defined in claim 1 by genetic recombination utilizing a nucleic acid according to either one of claims 2 or 3, or a vector according to claim 4.

7. Process for degradation of ulvans comprising a step of contacting ulvans with an ulvan lyase according to claim 1, or with a host cell according to claim 5, under conditions enabling the degradation of the ulvans by enzymatic digestion by said ulvan lyase or said host cell.

8. Use of an ulvan lyase as defined in claim 1 for the production of oligo-ulvans.
